# EUROPEAN PATENT SPECIFICATION

(11) **EP 3 962 597 B1**
(45) Date of publication and mention of the grant of the patent: **14.10.2026**
(21) Application number: 20731613.4
(22) Date of filing: 29.04.2020
(51) Int. Cl.: A61N 5/10

(54) **MULTILEAF COLLIMATOR CONE ENABLING STEREOTACTIC RADIOSURGERY**
MEHRBLATTKOLLIMATORKONUS FÜR STEREOTAKTISCHE RADIOCHIRURGIE
CÔNE DE COLLIMATEUR MULTILAME PERMETTANT UNE RADIOCHIRURGIE STÉRÉOTAXIQUE

(30) Priority: 30.04.2019 US 201916399828
(43) Date of publication of application: 09.03.2022
(73) Proprietor: Varian Medical Systems, Inc., Palo Alto, CA 94304 (US)
(72) Inventor: ZANKOWSKI, Corey, Palo Alto, California 94304 (US); MUTIC, Sasa, Palo Alto, California 94304 (US)
(74) Representative: Foster, Mark Charles
(86) International application number: PCT/US2020/030326
(87) International publication number: WO 2020/223266

(56) References cited:
- EP-A1- 1 720 173
- US-A1- 2015 273 239
- US-A1- 2019 091 489

## Description

### TECHNICAL FIELD

This disclosure relates generally to radiation apparatuses and methods. In particular, various embodiments of multileaf collimators (MLCs) enabling and enhancing radiosurgery and stereotactic radiosurgery are described.

### BACKGROUND

Radiosurgery is a highly precise, intensified form of radiation therapy. Stereotactic radiosurgery (SRS) is generally known as application of high doses of radiation in a single fraction based on specially defined treatment coordinates. Stereotactic radiotherapy is similar to SRS but with the exception that typically two or more fractions are used for patient treatments. It is worth noting that the term stereotactic may imply use of a stereotactic coordinate system for patient localization for treatment. Over time, image guided procedures and other forms of patient and tumor localization which do not rely on stereotactic coordinates have fallen in the category of stereotactic procedures even though by classical definitions the stereotactic localization method is not employed.

Conventionally, SRS cones are used to help achieve precise delivery of high dose of radiation. SRS cones are typically made from tungsten and have a conical hole through which radiation can pass providing a focused treatment beam. SRS cones can be mounted externally to an interface mount on a linear accelerator or internally on a positioning device in the treatment head. Inclusion of SRS cones in a treatment head requires more space for installation and complex motion axes. If mounted externally, SRS cones may present potential collision hazard with treatment couch or patient and generally involve compromises in efficiency of treatment delivery.

U.S. Patent Application Publication No. 2017-0197094 discloses a "virtual cone" approach using a multileaf collimator (MLC). According to the "virtual cone" approach, an MLC is used to form an actual elongated aperture through which radiation is delivered to a target volume at a first orientation of the MLC. Then, the MLC is rotated to a second orientation and radiation is delivered to the target volume through the actual elongated aperture. A relatively large cumulative dose of radiation can be thus delivered to the target volume through a "virtual cone" created by an area of overlap between the actual elongated apertures at the first and second MLC orientations. The "virtual cone" approach requires double passes of an MLC per couch angle and the general MLC leaf shape designed for other purposes may result in compromises in the shape of delivered radiation and may cause concerns of users that it may not produce robust implementation of radiation delivery.
US2019/091489A1 describes an adjustable collimator comprising a controller and two blade sets arranged opposite to each other; the blade sets comprise a plurality of blades, the controller drives the blades to move so as to form a first irradiation field through which a ray can pass; at least one blade in the blade set is a small irradiation field blade, and at least one irradiation field hole is provided on the small irradiation field blade; the controller is further used to drive the small irradiation field blade to move such that the irradiation field hole becomes a second irradiation field through which the ray can pass, wherein the second irradiation field is smaller than the first irradiation field.
US2015/273239A1 describes a multi level multileaf collimator that employs leaves with leaf tips having a non-square shape in a beam's eye view to improve beam shaping effect and penumbra performance. The multi level multileaf collimator includes a first multileaf collimator in a first level comprising beam blocking leaves longitudinally movable in a first direction, and a second multileaf collimator in a second level comprising beam blocking leaves longitudinally movable in a second direction. The first direction may be generally parallel with the second direction and the leaves of the first multileaf collimator may laterally offset the leaves of the second multileaf collimator. The beam blocking leaves of the first multileaf collimator may comprise an end portion having a non-square shape in a beam's eye view.
EP1720173A1 describes a collimator to limit a beam of energetic rays from a point source impinging on a processable object, especially for stereotactic conformational irradiation of tumours, comprises a scanning device having a drive and an iris diaphragm with at least three leaves with adjoining side surfaces enclosing equal angles and forming a radiation-limiting opening. A pushing movement along the side surfaces takes place through a highest to a lesser number of diaphragm leaves.

### SUMMARY

In accordance with a first aspect of the invention, there is provided an apparatus as defined by claim 1.
In accordance with a second aspect of the invention, there is provided a non-medical method of controlling a multi-level multileaf collimator as defined by claim 12.
Optional features are defined by the dependent claims.

As the beam exits the through-hole or aperture it may have a diameter or width that is less than the width of a leaf of the multileaf collimator as may advantageously be suitable for SRS, but it should be appreciated that the apparatus could be used for other applications.

The non-planar tip profile may have a recess that defines the space. The non-planar tip profile may have a bevelled edge that defines the space.

The spaces at the end portions of two adjacent pairs of the beam-blocking leaves are configured to collectively form the aperture when the two adjacent pairs of beam-blocking leaves are closed.

The aperture has a generally circular shape in a beam's eye view - e.g. formed by four generally quarter-circular recesses.

The aperture may have a generally truncated cone or cylindrical shape in a beam's eye view.

In one embodiment at least one of the beam-blocking leaves has a tip profile in a beam's eye view comprising a middle line section orthogonal to a direction in which the beam-blocking leaves are longitudinally moveable and a beveled line section at at least one side of the middle line section. A beveled line section may be provided either side of the middle line section. The beveled line section at the end portions of two adjacent pairs of the beam-blocking leaves may collectively form the a generally rectangular aperture when the two adjacent pairs of beam-blocking leaves are closed.

The said at least one of the beam-blocking leaves may have a chamfer angle formed between the beveled line and the middle line ranging from 20-80 degrees.

In one embodiment at least one of the beam-blocking leaves of the two adjacent pairs of the first collimator has a tip profile in the beam's eye view comprising a middle line section orthogonal to a direction in which the beam-blocking leaves are longitudinally moveable and a quarter-circular line section at at least one side of the middle line section. A quarter-circular line section may be provided either side of the middle line section. The quarter-circular line line section at the end portions of two adjacent pairs of the beam-blocking leaves may collectively form the a generally circular aperture when the two adjacent pairs of beam-blocking leaves are closed.

In one embodiment the first through-hole is provided in a first bank of said beam-blocking leaves arranged side by side, the first bank being opposite a second bank of beam-blocking leaves arranged side by side.

The first through-hole may have a generally truncated cone shape or cylindrical shape in a beam's eye view.

At least one of the plurality of beam-blocking leaves in the second bank may be provided with a second through-hole configured to allow a radiation beam to pass through for radiosurgery, wherein the second through-hole has a size different from a size of the first through-hole.

At least one of the plurality of beam-blocking leaves in the first bank may be further provided with a second through-hole configured to allow a radiation beam to pass through for radiosurgery, wherein the second through-hole has a size different from a size of the first through-hole.

The through-hole or the aperture may be disposed in or proximate to a middle of the plurality of beam blocking leaves the multileaf collimator to allow alignment of the through-hole or the aperture with a beam's central axis when in use.

The through-hole or the aperture may have a projected diameter of a size at an isocenter plane suitable for SRS.

In addition to the first multileaf collimator a second multileaf collimator comprising a plurality of beam-blocking leaves may be provided, wherein
the first multileaf collimator is arranged in a first level and the second multileaf collimator is arranged in a second level;
the beam-blocking leaves of the first multileaf collimator are longitudinally movable in a first direction, the beam-blocking leaves of the second multileaf collimator are longitudinally movable in a second direction generally parallel with the first direction; and
each of the beam-blocking leaves of the second multileaf collimator is laterally offset from a beam-blocking leaf of the first multileaf collimator in a beam's eye view.

The second multileaf collimator may be configured like the first multileaf collimator.

The at least one of the plurality of beam-blocking leaves of the second multileaf collimator may be provided with at least one of (i) a through-hole configured to provide a radiation passage to allow a radiation beam to pass through for radiosurgery and (ii) an end portion with a non-planar tip profile that defines a space configured to provide an aperture to allow a radiation beam to pass through for radiosurgery. The second multileaf collimator may have leaves configured in the same manner as the first multileaf collimator (but laterally offset therefrom) - e.g. to collectively form the aperture. The second multileaf collimator may have leaves configured in a different manner to the first multileaf collimator - e.g. to collectively form different shaped apertures in the first and second collimators. The second multileaf collimator may have leaves configured in a different manner to the first multileaf collimator - e.g. to provide a different through-hole size, or no through hole, in the second multileaf collimator.

In one embodiment at least two adjacent pairs of beam-blocking leaves in the first multileaf collimator have end portions configured to collectively form the aperture when the two adjacent pairs of beam-blocking leaves are closed, the method further comprising:
opening a pair of beam-blocking leaves in the second multileaf collimator overlaying the at least two adjacent pairs of beam-blocking leaves of the first multileaf collimator to form a second aperture in the second multileaf collimator, wherein the second aperture in the second multileaf collimator has a size greater than a size of the first aperture in the first multileaf collimator; and
closing the plurality of pairs of beam-blocking leaves of the first multileaf collimator, wherein the beam-blocking leaves of the at least two adjacent pairs of the first multileaf collimator partially block the second aperture in the second multileaf collimator, allowing the first aperture in the first multileaf collimator to control a size of a radiation beam passing through the multi-level multileaf collimator.

An apparatus is provided comprising a first multileaf collimator including a plurality of pairs of beam-blocking leaves each comprising an end portion. The end portions of beam-blocking leaves of two adjacent pairs are configured to collectively form an aperture when the two adjacent pairs of beam-blocking leaves are closed. The aperture has a generally circular shape in a beam's eye view.

A radiosurgery method, not forming part of the invention, using a multi-level multileaf collimator (MLC) is also described. The multi-level MLC comprises a first MLC having a plurality of pairs of beam-blocking leaves in a first level and a second MLC having a plurality of pairs of beam-blocking leaves in a second level. At least two adjacent pairs of beam-blocking leaves in the first MLC have end portions configured to collectively form a first aperture when the two adjacent pairs of beam-blocking leaves are closed. The method comprises opening a pair of beam-blocking leaves in the second MLC overlaying the at least two adjacent pairs of beam-blocking leaves of the first MLC to form a second aperture in the second MLC, where the second aperture in the second MLC has a size greater than the size of the first aperture in the first MLC; closing the plurality of pairs of beam-blocking leaves of the first MLC, whereby the beam-blocking leaves of the two adjacent pairs of the first MLC partially block the second aperture in the second MLC, allowing the first aperture in the first MLC to control the size and/or shape of a radiation beam passing through the multi-level MLC; and delivering the radiation beam to a target volume through the multi-level MLC, whereby the radiation beam delivered to the target volume is sized and shaped by the first aperture in the first MLC.

This Summary is provided to introduce selected aspects and embodiments of this disclosure in a simplified form and is not intended to identify key features or essential characteristics of the claimed subject matter, nor is it intended to be used as an aid in determining the scope of the claimed subject matter. The selected aspects and embodiments are presented merely to provide the reader with a brief summary of certain forms the invention might take and are not intended to limit the scope of the invention. Other aspects and embodiments of the disclosure are described in the section of Detailed Description.

### BRIEF DESCRIPTION OF THE DRAWINGS

These and various other features and advantages will become better understood upon reading of the following detailed description in conjunction with the accompanying drawings provided below, where:
FIG. 1 is a simplified illustration of a radiation system including a multileaf collimator (MLC) in accordance with embodiments of this disclosure;
FIG. 2 is a cross-sectional view of the exemplary MLC shown in FIG. 1, taken along line A-A.
FIG. 3A depicts exemplary beam-blocking leaves of two adjacent pairs where the leaves of each pair are opened or retracted showing the leaf tip profiles. FIG. 3B depicts exemplary beam-blocking leaves of two adjacent pairs where the leaves of each pair are closed forming an aperture.
FIGS. 4A-4C are bottom views of a multi-level MLC including beam-blocking leaves shown in FIGS. 3A-3B where all of the leaves are closed. FIG. 4A is a bottom view of the top MLC. FIG. 4C is a bottom view of the bottom MLC. FIG. 4B is a bottom view of the overlaid top and bottom MLCs.
FIGS. 5A-5C are top views of a multi-level MLC including beam-blocking leaves shown in FIGS. 3A-3B where all of the beam-blocking leaves are closed. FIG. 5A is a top view of the top MLC. FIG. 5C is a top view of the bottom MLC. FIG. 5B is a top view of the overlaid top and bottom MLCs.
FIGS. 6A-6C illustrate a method of method of controlling a multi-level MLC, that may be used to provide stereotactic radiosurgery (SRS), including beam-blocking leaves shown in FIGS. 3A-3B. FIG. 6A is a bottom view of the top MLC showing that a pair of beam-blocking leaves are opened. FIG. 6C is a bottom view of the bottom MLC showing that the beam-blocking leaves are closed. FIG. 6B is a bottom view of the overlaid top and bottom MLCs showing an aperture formed therein enabling SRS delivery.
FIGS. 7A-7C illustrate a method of method of controlling a multi-level MLC, that may be used to provide stereotactic radiosurgery (SRS), including beam-blocking leaves shown in FIGS. 3A-3B. FIG. 7A is a top view of the top MLC showing that a pair of beam-blocking leaves are opened. FIG. 7C is a top view of the bottom MLC showing that the beam-blocking leaves are closed. FIG. 7B is a top view of the overlaid top and bottom MLCs showing an aperture formed therein enabling SRS delivery.
FIG. 8A depicts exemplary beam-blocking leaves of two adjacent pairs where the leaves of each pair are opened or retracted showing the leaf tip profiles according to embodiments of the disclosure. FIG. 8B illustrates the beam-blocking leaves of the two adjacent pairs where the leaves of each pair are closed forming an aperture.
FIGS. 9A-9C illustrate a method of method of controlling a multi-level MLC, that may be used to provide stereotactic radiosurgery (SRS), including beam-blocking leaves shown in FIGS. 8A-8B. FIG. 9A is a top view of the top MLC showing that a pair of beam-blocking leaves are opened. FIG. 9C is a top view of the bottom MLC showing that the beam-blocking leaves are closed. FIG. 9B is a top view of the overlaid top and bottom MLCs showing an aperture formed therein enabling SRS delivery.
FIGS. 10A-10C illustrate a method of method of controlling a multi-level MLC, that may be used to provide stereotactic radiosurgery (SRS), including beam-blocking leaves shown in FIGS. 8A-8B. FIG. 10A is a bottom view of the top MLC showing that a pair of beam-blocking leaves are opened. FIG. 10C is a bottom view of the bottom MLC showing that the beam-blocking leaves are closed. FIG. 10B is a bottom view of the overlaid top and bottom MLCs showing an aperture formed therein enabling SRS delivery.
FIG. 11 depicts an exemplary MLC comprising beam-blocking leaves arranged in two opposing banks.
FIG. 12 depicts an exemplary MLC according to embodiments of the disclosure where a through-hole is provided in a beam-blocking leaf in a bank.
FIG. 13 depicts an exemplary MLC according to embodiments of the disclosure where two through-holes of different sizes are provided in a beam-blocking leaf in a bank.
FIG. 14 depicts an exemplary MLC according to embodiments of the disclosure where a first through-hole is provided in a beam-blocking leaf in a bank and a second through-hole of a different size is provided in a beam-blocking leaf in the opposing bank.

### DETAILED DESCRIPTION OF EMBODIMENTS

Embodiments of this disclosure provide specially designed multileaf collimators (MLCs) and solutions for controlling a an MLC, that may be used to provide radiosurgery and stereotactic radiosurgery. One exemplary approach is to configure or modify the MLC leaf tips so that they form a small cone when the MLC leaves are closed and/or are more conducive to cone-like profiles than are conventional MLCs with "virtual cone." Another exemplary approach is to provide one or more through-holes in one or more MLC leaves. The solutions provided by this disclosure enable delivery of small SRS cone-like beam profiles directly from an MLC while fully preserving the general purpose or functionality of the MLC. The multileaf collimators of the embodiments may have other medical and non-medical applications. The multileaf collimators may be used to shape radiation beams for many purposes, including non-medical purposes.

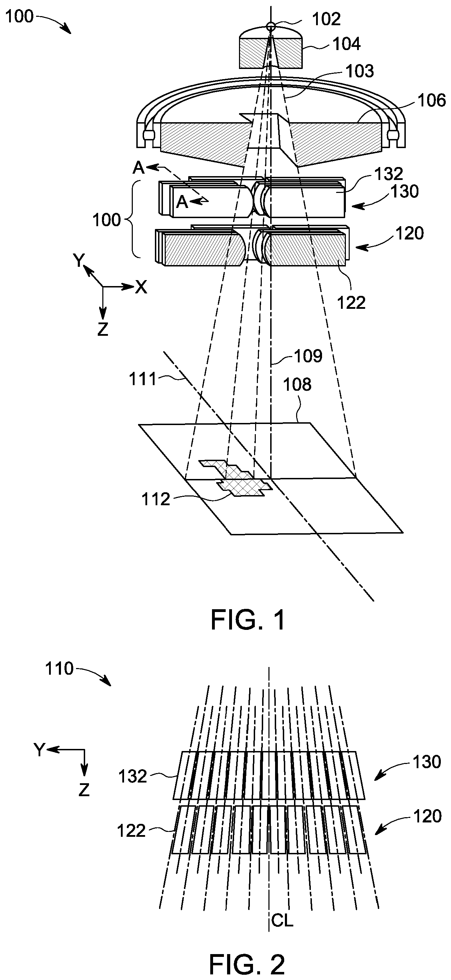

Referring to FIGS. 1-14, various embodiments of multileaf collimators (MLCs) that are, for example, useable to enable and enhance radiosurgery and stereotactic radiosurgery (SRS) will now be described.

FIG. 1 is a simplified illustration of a radiation system including an MLC according to embodiments of the disclosure. As shown, the radiation system 100 may include a radiation source 102 configured to produce a beam 103 of radiation such as photons, electrons, protons, or other types of radiation. For example, the radiation source 102 may include a metallic target configured to produce a beam of x-rays upon impingement of electrons. The radiation system 100 may include various beam shaping components such as a primary collimator 104 and optionally a secondary collimator 106 to generally limit the extent of the beam 103 as it travels away from the source 102 toward an isocenter plane 108. An MLC 110 such as a multi-level MLC is disposed between the source 102 and the isocenter plane 108 to further shape the beam, as indicated by the shaped field 112 in the isocenter plane 108, according to a general use embodiment of the MLC. The MLC 110 may rotate about the beamline or axis 109 passing through the source 102, placing the MLC in various orientations. The source 102, primary collimator 104, secondary collimator 106, and the MLC 110 may be enclosed in a treatment head (not shown), which can be rotated by a gantry (not shown) about an axis such as a horizontal axis 111. Thus, the radiation system 100 can deliver treatment beams to a target in the isocenter plane 108 from various angles. The shape, size, and/or intensity of the beam 103 can be adjusted or dynamically adjusted by the MLC 110 as the beam angle is stepped or swept around the target.

The MLC 110 may be a single level MLC or a multi-level MLC as shown. By way of example, the MLC 110 may include a first MLC 120 in a first level distal to the source 102 and a second MLC 130 in a second level proximal to the source 102. As used herein, the term "multileaf collimator" or "MLC" refers to a collection of a plurality of beam-blocking leaves each of which can be longitudinally moved in and out of a beam to modify one or more parameters of the beam such as the beam shape, size, energy, or intensity etc. Each beam-blocking leaf may be driven by a motor with a lead screw or other suitable means. The beam-blocking leaves may be arranged in pairs. The beam-blocking leaves of each pair may be brought in contact or retracted from each other to close or open a path for a radiation beam to pass through the MLC. The beam-blocking leaves are arranged in opposing banks and may be supported by a frame, box, carriage or other support structure, which has features allowing the individual beam-blocking leaves to extend into and retract from the beam. The frame, box, carriage or other support structure can be further moved or translated in addition to the individual leaf travel.

As shown in FIG. 1, the first and second MLCs 120, 130 may be arranged such that the moving direction of individual beam-blocking leaves of the first and second MLCs 120, 130 are generally in parallel. As shown in FIG. 1 the beam-blocking leaves 122 of the first MLC 120 in the first level are longitudinally movable in the x-direction, and the beam-blocking leaves 132 of the second MLC 130 in the second level are also longitudinally movable in the x-direction. Alternatively, the first and second MLCs may be arranged such that the moving direction of the beam-blocking leaves of the first MLC is non-parallel e.g. perpendicular to the moving direction of the beam-blocking leaves of the second MLC.

The first and second MLCs 120, 130 may be arranged such that the leaves 132 of the second MLC 130 may laterally offset the leaves 122 of the first MLC 120 in a beam's eye view, or as viewed in a direction from the source 102. FIG. 2 is a cross-sectional view of a portion of the multi-level MLC 110 of FIG. 1 taken along line A-A, showing the lateral offset arrangement of the leaves of the multi-level MLC 110. As shown, a leaf 132 of the second MLC 130 in the second level offsets a leaf 122 of the first MLC 120 in the first level as viewed from the source 102. By way of example, a leaf 132 of the second MLC 130 may offset a leaf 122 of the first MLC 120 by substantially half a leaf. Alternatively, a gap between two adjacent leaves 132 of the second MLC 130 in the second level may be positioned substantially at the middle of a leaf 122 of the first MLC 120. The lateral offset arrangement of leaves in different levels provides for leaf projections that are also offset at the isocenter plane. Therefore, the lateral offset arrangement of leaves may provide for substantially an equivalent of doubling MLC definition, or improving the resolution to half as compared to the definition of a single level MLC with leaves of the same physical width. In some embodiments, three or more MLCs may be arranged in three or more levels such that each leaf at a level may offset e.g. by 1/3 or 1/n of a leaf width as projected at the isocenter plane where n is the number of the MLCs. U.S. Pat. No. 8,637,841 issued on Jan. 28, 2014 to the common assignee entitled "Multi Level Multileaf Collimators" describes various embodiments of multi-level MLCs.

The beam-blocking leaves 122, 132 of the first and second MLCs 120, 130 may have various leaf tip profiles or end portion configurations. For ease of description of the leaf tip profiles and the MLC in general, in the Detailed Description and appended Claims, the term "top view" may be used interchangeably with the term "beam's eye view" to refer to a view observed from the source or in a direction parallel to the beamline. The term "bottom view" may be used to refer to a view opposite to the top view of the leaf tip profile. The term "side view" may be used to describe a view observed from a side surface of the leaf tip profile.

In some embodiments, the beam-blocking leaves of the MLC 110 may have a flat front-end surface. In both a side view and a beam's eye view, a leaf tip with a flat (or planar) front-end surface may be shown to have a straight line orthogonal to the leaf longitudinal moving direction and two right angles at each side of the straight line. In some embodiments, the beam-blocking leaves of the MLC 110 may have a curved front-end surface. In a side view, a leaf with a curved front-end surface may be shown to have a curved line with a radius and two parallel lines at either side of the curved line. In a top view, a leaf with a curved front-end surface may be shown to have a straight line orthogonal to the leaf longitudinal moving direction and two right angles on each side of the straight line. In the Detail Description and appended Claims, the term "square shape" may be used to describe a leaf tip profile which, in a top view, has a straight line orthogonal to the leaf longitudinal moving direction and two right angles at each side of the straight line. The term "non-square shape" may be used to describe any leaf tip profile which, in a top view, does not have a square shape. The non-square shape in a top view may include a curved or elliptic shape or a shape of a chamfer leaf which may include a combination of a straight portion and a beveled portion at each side of the straight portion as will be described in greater detail below.

In some embodiments of this disclosure, the beam-blocking leaves of the MLC 110 may have a tip profile that includes a combination of a curved surface portion and a beveled or flat surface portion at each side of the curved surface portion. The term "chamfer leaf" may be used herein to refer to a leaf which includes a combination of a curved end surface portion and beveled or flat end surface portions at either side of the curved end surface portion.

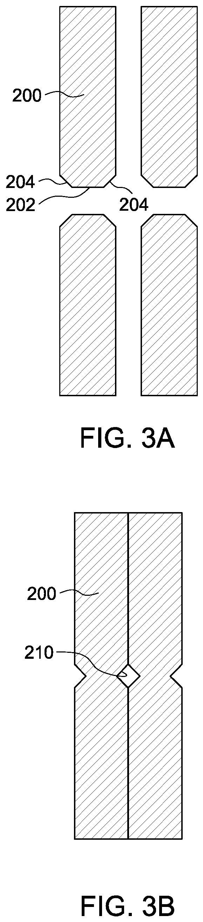

FIGS. 3A-3B are top views of exemplary beam-blocking leaves 200 according to embodiments of this disclosure. FIG. 3A shows two adjacent pairs of beam-blocking leaves 200 when the leaves of each pair are retracted or opened. It should be noted that in FIG. 3A, the gap between the sides of the adjacent pairs of the leaves is exaggerated for purpose of illustrating the leaf tip profiles with greater clarity. FIG. 3B shows two adjacent pairs of beam-blocking leaves 200 when the leaves of each pair are brought into contact or closed forming an aperture 210.

As shown in FIG. 3A, in a top view or beam's eye view, the end portion or tip of a beam-blocking leaf 200 may be shown to have a straight middle line section 202 orthogonal to the leaf longitudinal moving direction and beveled or angled lines sections 204 at each side of the straight middle line section 202. The term "chamfer angle" may be used herein to refer to the angle between the beveled line 204 and the straight line 202. For example, the straight middle line section 202 of a leaf in a beam's eye view may be approximately 50% of the leaf width, and the remaining 25% at each side of the middle section can be shaped in any different chamfer angles optimized for various different field slopes. The chamfer angles may range from 5-95 degrees, or from 10-90 degrees, or from 20-80 degrees, or from 40-60 degrees. In one example, the chamfer angle may be about 45 degrees. In another example, the chamfer angle may be about 60 degrees. In a specific embodiment, a beam-blocking leaf may have a straight middle line section about 50% of the leaf width, with the remaining 25% at each side being beveled with a chamfer angle of about 45 or 60 degrees.

As shown in FIG. 3B, when two adjacent pairs of beam-blocking leaves 200 are brought in contact or closed, an aperture 210 is formed collectively by the end portions of four adjacent beam-blocking leaves 200. The aperture 210 may extend from the top of the leaves to the bottom of the leaves or the height of the leaves forming a path for a radiation beam. The size and/or shape of the aperture 210 may be defined by the tip profile of the beam-blocking leaves 200. For example, the chamfer angle, the leaf thickness, and the ratio of the beveled section to the middle section etc. may be selected so that the aperture 210 formed may have a size and shape suitable for SRS delivery. By way of example, the aperture 210 formed by the beam-blocking leaves 200 when closed may have a rectangular or square shape in a top view as shown in FIG. 3B or an effective rectangular prism shape extending from the top to the bottom of the leaves. The side of the square of the aperture 210 is therefore smaller than the thickness of the individual beam-blocking leaves 200. By way of example, the aperture 210 may have a square shape in a top view having a side dimension ranging from 2 to 10 millimeter.

It should be understood that, in a top view or beam's eye view, the end portion or tip of a beam-blocking leaf 200 may have a straight middle line section 202 orthogonal to the leaf longitudinal moving direction and only a single beveled or angled line sections 204. The single beveled or angled line sections 204 may be at one side of the straight middle line section 202.

It should be understood that, in a top view or beam's eye view, the end portion or tip of a beam-blocking leaf 200 may have a straight line section 202 orthogonal to the leaf longitudinal moving direction and one or more recesses in the straight line section 202. The recess may be curved (e.g. semi-circular or semi-elliptical), or have two or more straight sides.

In general, in some embodiments, one or more of the beam-blocking leaves 200 has a non-planar tip profile that defines a space configured to allow a radiation beam to pass through for radiosurgery. The space may be one or more beveled or angled line sections 204 or one or more recesses in the straight line section 202.

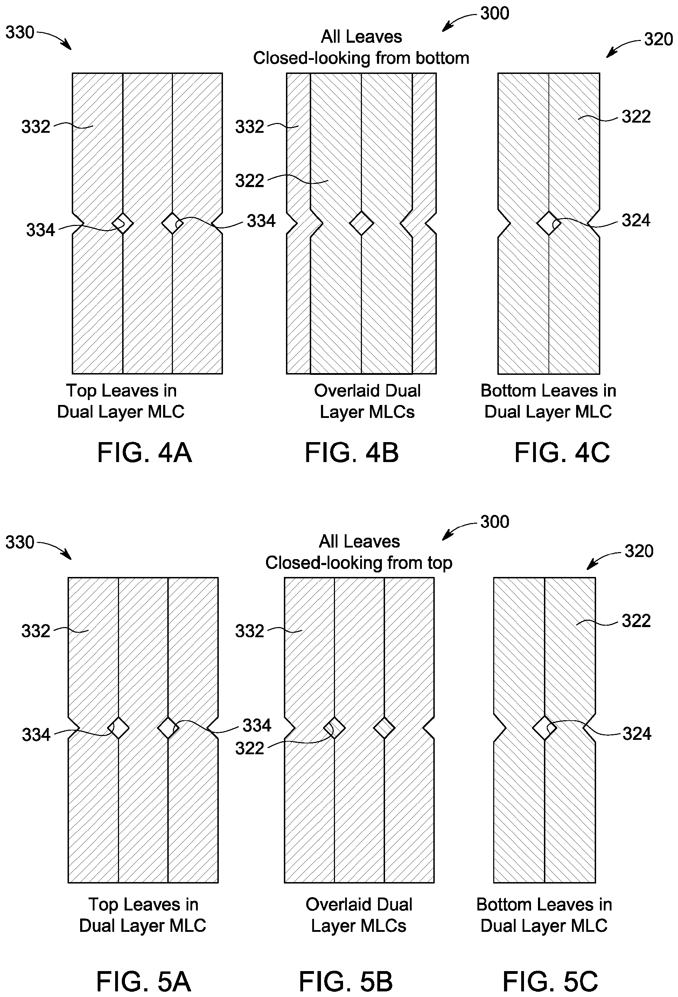

FIGS. 4A-4C, 5A-5C, 6A-6C, and 7A-7C illustrate an embodiment of a method of controlling a multi-level MLC 300 that may be used to provide stereotactic radiosurgery (SRS). The multi-level MLC 300 may include a first or bottom MLC 320 and a second or top MLC 330 comprising beam-blocking leaves shown in FIGS. 3A-3B (or one of the alternative forms of beam-blocking leaves mentioned above). The bottom and top MLCs 320, 330 can be arranged such that the longitudinal moving direction of the beam-blocking leaves 322 of the bottom MLC 320 is generally parallel with the longitudinal moving direction of the beam-blocking leaves 332 of the top MLC 330. The first and second MLCs 320, 330 can be arranged such that the beam-blocking leaves 332 of the top MLC 330 laterally offset the beam-blocking leaves 322 of the bottom MLC 320 e.g. by about half or a third of a leaf width. For clarity, only three pairs of beam-blocking leaves 332 in the top MLC 330 and only two pairs of beam-blocking leaves 322 in the bottom MLC 320 are shown. It should be noted that the bottom and top MLC 320, 330 may include more than two or three pairs of beam-blocking leaves.

FIGS. 4A-4C are bottom views of the MLCs 300. FIG. 4A is a bottom view of the top MLC 330, FIG. 4C a bottom view of the bottom MLC 320, and FIG. 4B a bottom view of the overlaid top and bottom MLCs 300. As shown in FIG. 4A, the use of beam-blocking leaves 332 in the top MLC 330 results in apertures 334 when all the leaves 332 are closed. Similarly, as shown in FIG. 4C, the use of beam-blocking leaves 322 in the bottom MLC 320 results in aperture 324 when all the leaves are closed. Nevertheless, because of the lateral-offset arrangement of the top and bottom MLCs 330, 320, the apertures 334 in the top MLC 330 are blocked by the beam-blocking leaves 322 of the bottom MLC 320, and the aperture 324 in the bottom MLC 320 is blocked by the beam-blocking leaves 332 of the top MLC 330, as shown in FIG. 4B. Therefore, when all beam-blocking leaves 332 of the top MLC 330 and all beam-blocking leaves 322 of the bottom MLC 320 are closed, no beam path is formed in the multi-level MLC 300 as shown in FIG. 4B. The leaves 322 and 332 may be moved with respect to another to shape a radiation beam in a similar manner to that shown in Figure 1. By moving the leaves of the bottom MLC 320 and of the top MLC 330 such that any part of the aperture 334 in the leaves of the top MLC 330 never overlies any part of the aperture 324 in the leaves of the bottom MLC 320 the apertures 324 and 334 do not allow the passage of radiation through the multi-layer MLC at the apertures 324 and 334, accomplishing a general functionality of the MLC - for example as shown in Figure 1. The MLC of the embodiment can therefore be used in a SRS delivery mode or a general MLC mode.

FIGS. 5A-5C are top views of the MLCs 300. FIG. 5A is a top view of the top MLC 330, FIG. 5C a top view of the bottom MLC 320, and FIG. 5B a top view of the overlaid top and bottom MLCs 300. As shown in FIG. 5A, the use of beam-blocking leaves 332 in the top MLC 330 result in apertures 334 when all the beam-blocking leaves 332 are closed. Similarly, as shown in FIG. 5C, the use of beam-blocking leaves 322 in the bottom MLC 320 result in aperture 324 when all the beam-blocking leaves 322 are closed. Nevertheless, because of the lateral-offset arrangement of the top and bottom MLCs 330, 320, the apertures 334 in the top MLC 320 are blocked by the beam-blocking leaves 322 of the bottom MLC 320, and the aperture 324 in the bottom MLC 320 is blocked by the beam-blocking leaves 332 of the top MLC 300, as shown in FIG. 5B. Therefore, when all beam-blocking leaves 332 of the top MLC 330 and all beam-blocking leaves 322 of the bottom MLC 320 are closed, no beam path is formed in the multi-level MLC 300 as shown in FIG. 4B. The leaves 322 and 332 may be moved with respect to another to shape a radiation beam in a similar manner to that shown in Figure 1. By moving the leaves of the bottom MLC 320 and of the top MLC 330 such that any part of the aperture 334 in the leaves of the top MLC 330 never overlies any part of the aperture 324 in the leaves of the bottom MLC 320 the apertures 324 and 334 do not allow the passage of radiation through the multi-layer MLC the apertures 324 and 334, accomplishing a general functionality of the MLC - for example as shown in Figure 1.

The general functionality of the MLC, can also be provided with other leaf configurations. For example, this can be achieved where one or more of the beam-blocking leaves 200 has a non-planar tip profile that defines a space configured to allow a radiation beam to pass through for radiosurgery (the space may be one or more beveled or angled line sections 204 or one or more recesses in the straight line section 202). The width of the space (measured in a direction orthogonal to the leaf longitudinal movement direction) may be less than or equal to the width of the straight line section 202. This allows blocking of the beam path if there are two laterally offset MLCs (a two-level MLC). If there are more than two laterally offset MLCs the width of the space may be correspondingly reduced to provide blocking of the beam path.

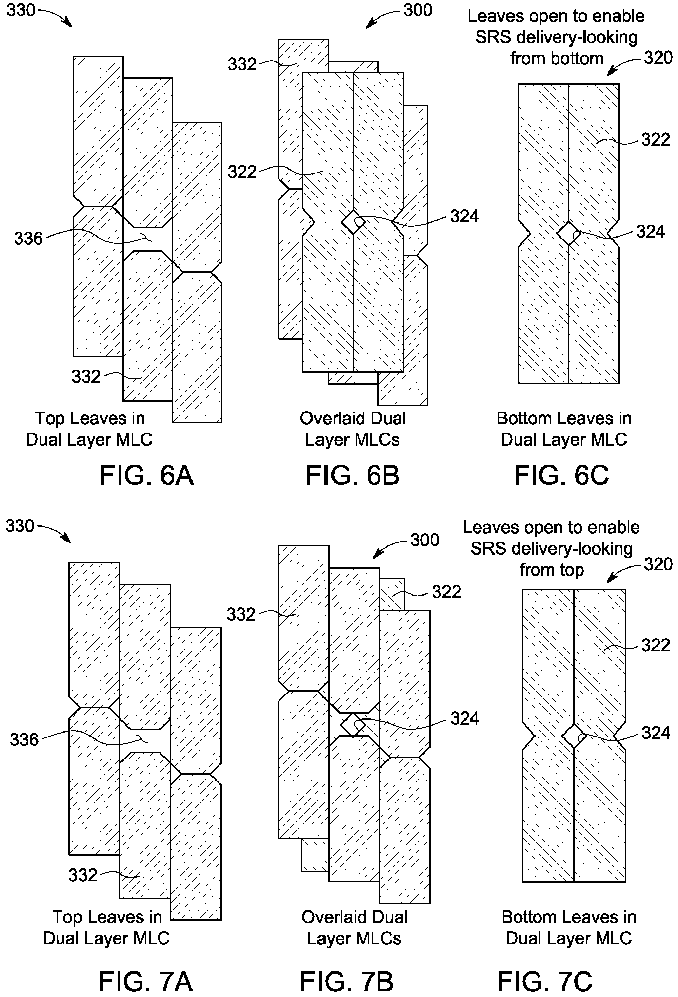

FIGS. 6A-6C are bottom views of the top MLC 330 (FIG. 6A), bottom MLC 320 (FIG. 6C), and overlaid top and bottom MLCs 300 (FIG. 6B) illustrating a method of controlling a multi-level MLC according to an embodiment of the disclosure, that may be used to provide stereotactic radiosurgery (SRS). MLCs 330 and 320 may include beam-blocking leaves shown in FIGS. 3A-3B. According to the embodiment of the method, a pair of beam-blocking leaves 332 in the top MLC 330 can be retracted or opened, forming an aperture 336 of a greater size in the top MLC 330, as shown in FIG. 6A. The beam-blocking leaves 322 in the bottom MLC 320 can be closed, resulting in an aperture 324 due to the use of beam-blocking leaves shown in FIGS. 3A-3B. FIG. 6B shows that the aperture 324 in the bottom MLC 320 is exposed to the aperture 336 of greater size 336 in the top MLC 330. The retraction or opening of the pair of beam-blocking leaves 332 in the top MLC 330 is such that the aperture 336 overlies the aperture 324. The lateral-offset arrangement of the top and bottom MLCs 330, 320 allows the beam-blocking leaves 322 of the bottom MLC 320 to partially block the aperture 336 in the top MLC 330. Therefore, when a pair of beam-blocking leaves 332 in the top MLC 330 is opened and all the other leaves in the top and bottom MLCs 330, 320 are closed, the aperture 324 in the bottom MLC 320 is exposed, forming a path to allow a radiation beam passing through the multi-level MLC 300. The size and shape of the aperture 324 control the size and shape of the radiation beam, enabling SRS delivery.

FIGS. 7A-7C are top views of the top MLC 330 (FIG. 7A), bottom MLC 320 (FIG. 7C), and overlaid top and bottom MLCs 300 (FIG. 7B) illustrating the method of method of controlling a multi-level MLC which has been described above in conjunction with the bottom views of FIGS. 6A-6C. For completeness, a pair of beam-blocking leaves 332 in the top MLC 330 can be retracted or opened, forming an aperture 336 of a greater size in the top MLC 330, as shown in FIG. 7A. The beam-blocking leaves 322 in the bottom MLC 320 can be closed, resulting in an aperture 324 due to the use of beam-blocking leaves shown in FIGS. 3A-3B. FIG. 7B shows that the aperture 324 in the bottom MLC 320 is exposed to the aperture 336 of greater size 336 in the top MLC 330. The lateral-offset arrangement of the top and bottom MLCs 330, 320 allows the beam-blocking leaves 322 of the bottom MLC 320 to partially block the aperture 336 in the top MLC 330. Therefore, when a pair of beam-blocking leaves 332 in the top MLC 330 is opened and all the other leaves in the top and bottom MLCs 330, 320 are closed, the aperture 324 in the bottom MLC 320 is exposed, forming a path to allow a radiation beam passing through the multi-level MLC 300. The size and shape of the aperture 324 control the size and shape of the radiation beam, enabling SRS delivery. The same principles apply, in general, when one or more of the beam-blocking leaves 200 has a non-planar tip profile that defines a space configured to allow a radiation beam to pass through for radiosurgery. The space may be one or more beveled or angled line sections 204 or one or more recesses in the straight line section 202.

In the method illustrated in FIGS. 6A-6C and 7A-7C, the aperture 324 formed in the multilevel MLC 300 can be aligned with the beam's central axis. By way of example, when in use, a pair of beam-blocking leaves in the middle or proximate to the middle of the top MLC 330 can be retracted as shown in FIGS. 6A and 7A, allowing the aperture 324 in the bottom MLC 320 to be aligned with the beam's central axis. The support structure of the MLC may also be moved relative to the source in aligning the aperture with the beam's central axis. It should be noted that the capability of aligning the aperture 324 with the beam's central axis, while preferred, is not required. The aperture 324 can be placed off the beam's central axis as long as it is within the beam divergence. A patient support or couch can be moved to align the target in the patient to be treated with the focused radiation beam passing through the aperture.

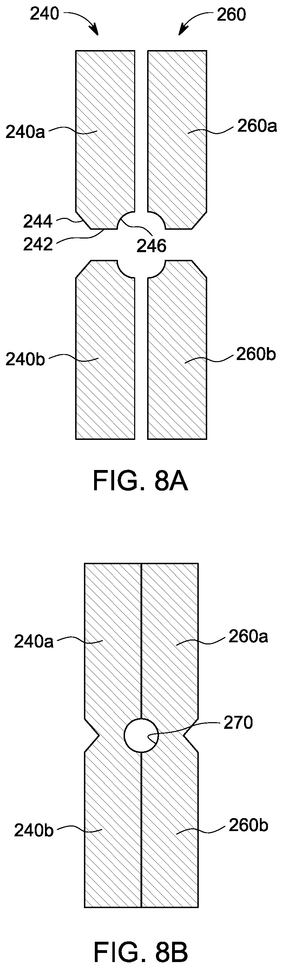

In some embodiments of the disclosure, an MLC may comprise two adjacent pairs of beam-blocking leaves having leaf tip profiles shown in FIGS. 8A-8B. The leaf tips or end portions of the beam-blocking leaves can be configured such that an aperture having a generally truncated cone shape or cylindrical shape or an equivalent of truncated cone or cylindrical shape is formed when the beam-blocking leaves are closed. In a top view or beam's eye view, the aperture formed may have a circular shape.

Referring to FIGS. 8A-8B, for example, leaf 240a of pair 240 may have a leaf tip profile including a concave surface portion extending from the top to the bottom of the leaf or the height of the leaf. In a beam's eye view, the concave surface portion will be shown to have a curved line section 246, as shown in FIG. 8A. Therefore, the end portion of leaf 240a may be configured so that in a beam's eye view, the tip of leaf 240a may be shown to have a straight middle line section 242 perpendicular to the leaf longitudinal moving direction, a beveled line section 244 at one side of the middle line section, and a curved line section 246 at another side of the middle line section. In a specific embodiment, the curved line section 246 may constitute a quarter-circle line. It should be understood that the side section 244 may be curved, rather than beveled - and so have a shape similar to the curved line section 246 but oppositely orientated.

Leaf 240b of pair 240 may have a leaf tip profile that is a mirror image of the leaf tip profile of the opposing leaf 240a. Therefore, when the leaves 240a, 240b of pair 240 are closed, a semi-circle line can be formed by the quarter-circle lines of leaves 240a and 240b in a beam's eye view, as illustrated in FIG. 8B. Likewise, leaf 260b of the adjacent pair 260 may have a leaf tip profile that is a mirror image of the leaf tip profile of opposing leaf 260a so that when the leaves 260a, 260b of the adjacent pair 260 are closed, a semi-circle line is formed by the quarter-circle lines of leaves 260a and 260b. When the two adjacent pairs of four beam-blocking leaves 240a, 240b and 260a, 260b are closed, a circle is formed by the quarter-circle lines of leaves 240a, 240b and 260a, 260b in the beam's eye view.

In some embodiments, the concave surface portions of the beam-blocking leaves 240a, 240b and 260a, 260b of the two adjacent pairs can be configured so that the aperture 270 formed when the beam-blocking leaves are closed has a generally cylindrical shape. In some embodiments, the concave surface portions of the beam-blocking leaves 240a, 240b and 260a, 260b of the two adjacent pairs can be configured so that the aperture 270 formed when the beam-blocking leaves are closed has a generally truncated cone shape. In some embodiments, the front-end portions of the beam-blocking leaves 240a, 240b and 260a, 260b of the two adjacent pairs can be configured so that the aperture 270 formed when the beam-blocking leaves are closed projects a circular shape on the isocenter plane, providing an equivalent of a truncated cone or cylindrical hole.

The pairs of beam-blocking leaves 240a-b, 260a-b shown in FIGS. 8A-8B may be disposed in the middle or proximate to the middle of the plurality of beam-blocking leaves of the MLC. Such arrangement may facilitate alignment of the aperture 270 with the beam's central axis when in use. Alternatively, the pairs of beam-blocking leaves 240a-b, 260a-b shown in FIGS. 8A-8B may not be disposed at the middle of the MLC leaves. The alignment with the aperture 270 can be achieved by moving the support structure of the MLC or moving the patient support or couch.

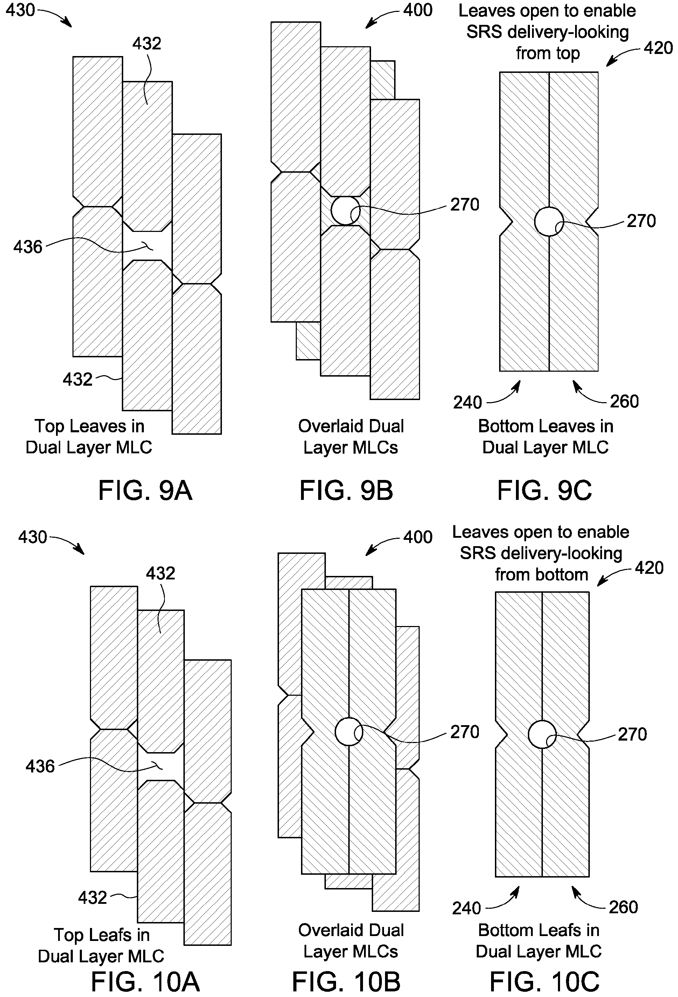

FIGS. 9A-9C and 10A-10C illustrate an embodiment of a method of method of controlling a multi-level MLC 400 according to an embodiment of the disclosure, that may be used to provide stereotactic radiosurgery (SRS). The multi-level MLC 400 comprises a first or bottom MLC 420 and a second or top MLC 430. The bottom MLC 420 may comprise two adjacent pairs of the beam-blocking leaves having leaf tip profiles as shown in FIGS. 8A-8B. The remaining pairs of the beam-blocking leaves of the bottom and top MLCs may have leaf tip profiles as shown in FIGS. 3A-3B (or some or all may have leaf tip profiles similar to the curved line section 246). The bottom MLC 420 and top MLC 430 may be arranged such that the longitudinal moving direction of the beam-blocking leaves of the bottom MLC 420 is generally parallel with the longitudinal moving direction of the beam-blocking leaves of the top MLC 430. The bottom and top MLCs 420, 430 can be arranged such that the beam-blocking leaves of the top MLC 430 laterally offset the beam-blocking leaves of the bottom MLC 420 e.g. by about half or a third of a leaf width. For clarity, only three pairs of beam-blocking leaves in the top MLC 430 and only two pairs of beam-blocking leaves in the bottom MLC 420 are shown. It should be noted that the bottom and top MLC 420, 430 may include more than two or three pairs of beam-blocking leaves.

FIGS. 9A-9C are top views of the top MLC 430 (FIG. 9A), bottom MLC 420 (FIG. 9C), and overlaid top and bottom MLCs 400 (FIG. 9B). FIGS. 10A-10C are bottom views of the top MLC 430 (FIG. 10A), bottom MLC 420 (FIG. 10C), and overlaid top and bottom MLCs 400 (FIG. 10B). According to an embodiment of the method, a pair of beam-blocking leaves 432 in the top MLC 430 can be retracted or opened, forming an aperture 436 of a greater size in the top MLC 430, as shown in FIGS. 9A and 10A. The beam-blocking leaves 240, 260 in the bottom MLC 420 can be closed, resulting in an aperture 270 due to the use of the beam-blocking leaves 240a, 240b, 260a, and 260b as shown in FIGS. 8A-8B. The aperture 270 may have a shape of a circle in a top or bottom view as shown in FIGS. 9C and 10C. FIGS. 9B and 10B show that the aperture 270 in the bottom MLC 420 is exposed to the aperture 436 of greater size in the top MLC 430. The retraction or opening of the pair of beam-blocking leaves 432 in the top MLC 430 is such that the aperture 436 overlies the aperture 270. The lateral-offset arrangement of the top and bottom MLCs 430, 420 allows the beam-blocking leaves 240, 260 of the bottom MLC 420 to partially block the aperture 436 in the top MLC 430. Therefore, when a pair of beam-blocking leaves 432 in the top MLC 430 is opened and all the other leaves in the top and bottom MLCs 430, 420 are closed, the aperture 270 in the bottom MLC 420 is exposed, forming a path to allow a radiation beam passing through the multi-level MLC 400. The aperture 270 controls the size and shape of the radiation beam, enabling SRS delivery.

In a similar manner as described in relation to Figures 4A-C and Figures 5A-C, the leaves 240, 260 in the bottom MLC 420 and the leaves 432 in the top MLC 430 may be moved with respect to another to shape a radiation beam in a similar manner to that shown in Figure 1. By moving the leaves of the bottom MLC 420 and of the top MLC 430 such that any part of the aperture 436 in the leaves of the top MLC 430 never overlies any part of the aperture 270 in the leaves of the bottom MLC 420 the apertures 270 and 426 do not allow the passage of radiation through the multi-layer MLC at the apertures 270 and 426, accomplishing a general functionality of the MLC - for example as shown in Figure 1. The MLC of the embodiment can therefore be used in a SRS delivery mode or a general MLC mode.

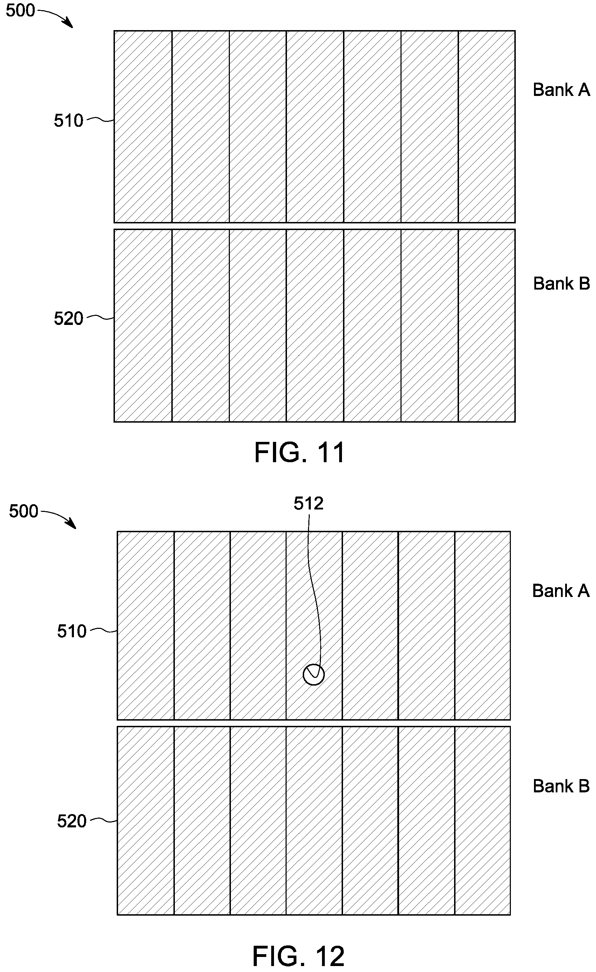

Referring now to FIGS. 11-14, alternative embodiments of multileaf collimators of the disclosures are now described. FIG. 11 depicts a multileaf collimator 500 comprising a plurality of beam-blocking leaves 510 arranged side by side in a first bank (Bank A) and a plurality of beam-blocking leaves 520 arranged side by side in a second bank (Bank B) opposite to the first bank (Bank A). Each of the plurality of beam-blocking leaves 510, 520 in the first and second banks is longitudinally movable.

According to embodiments of the disclosure, a beam-blocking leaf in the first bank may be provided with a first through-hole 512 as shown in FIG. 12. The first through-hole 512 may be sized and shaped, or configured, to allow a radiation beam to pass through the MLC 500 when in use for stereotactic radiosurgery. By way of example, the first through-hole 512 may have a generally truncated cone shape, a generally cylindrical shape, or any other regular or irregular shapes. The size of the first through-hole in a cross-section may range from 2 to 10 millimeters, 4 to 8 millimeters, or any other dimensions generally suitable for stereotactic radiosurgery. In a specific embodiment, the first through-hole may be sized so that a circle with a diameter of about 4 or 5 millimeters may be projected on the isocenter plane. As used herein, the term "about" includes variances within 1 millimeter of the size referred to. The beam-blocking leaf provided with the first through-hole 512 may be disposed in the middle or proximate to the middle of the plurality of beam-blocking leaves in the first bank (Bank A), as shown in FIG. 12. Such arrangement can facilitate alignment of the first through-hole with the beam's central axis when in use. For example, when in use the beam-blocking leaf provided with the first through-hole 512 may be longitudinally moved or extended to align the center of the through-hole with the beam's central axis. The support structure of the MLC may also be moved relative to the source in aligning the through-hole with the beam's central axis. It should be noted that the capability of aligning the through-hole with the beam's central axis, while preferred, is not required. In some embodiments, the beam-blocking leaf provided with a through-hole may not be disposed at the middle of the beam-blocking leaves of the MLC. Further, the through-hole can be placed off the beam's central axis as long as it is within the beam divergence when in use. A patient support or couch can be moved to align the target in the patient to be treated with the focused radiation beam passing through the through-hole.

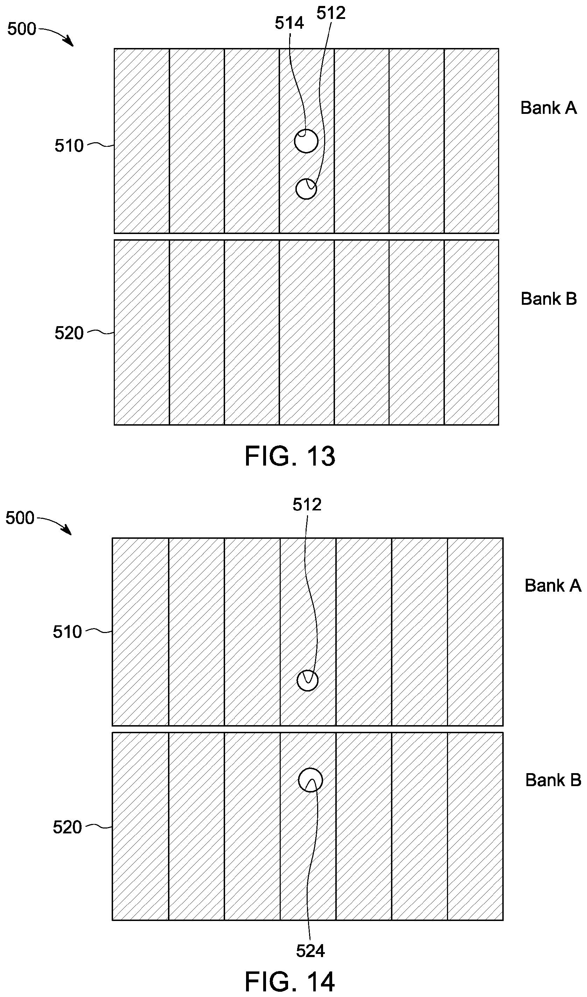

In some embodiments, the beam-blocking leaf provided with the first through-hole 512 may be further provided with a second through-hole 514 as shown in FIG. 13. The second through-hole 514 may be configured to allow a radiation beam to pass through the MLC 500 when in use for stereotactic radiosurgery. The size of the second through-hole 514 may be different from or same as the size of the first through-hole 512. The shape of the second through-hole 514 may be the same or different from the shape of the first through-hole 512. FIG. 13 shows an embodiment where the first and the second through-holes 512, 514 are provided in a same beam-blocking leaf in a bank. Alternatively, the first and second through-holes may be provided in different beam-blocking leaves in a same bank.

According to alternative embodiments of the disclosure, a beam-blocking leaf in the first bank (Bank A) is provided with a first through-hole 512 and a beam-blocking leaf in the second bank (Bank B) is provided with a second through-hole 524, as shown in FIG. 14. The beam-blocking leaves provided with the first and second through-holes 512, 524 may be paired and longitudinally movable relative to each other. Alternatively, the beam-blocking leaves provided with the first and second through-holes are not paired. The first and second through-holes 512, 524 may be each configured to allow a radiation beam to pass through the MLC 500 when in use for stereotactic radiosurgery. The size of the second through-hole 524 may be different from or same as the size of the first through-hole 512 in the opposing beam-blocking leaf. The shape of the second and first through-holes 512, 524 in the opposing beam-blocking leaves may be same or different. By way of example, the second and first through-holes may have a generally truncated cone shape, a generally cylindrical shape, or any other regular or irregular shapes respectively. The size of the second and first through-holes in a cross-section may range from 2 to 10 millimeters, 4 to 8 millimeters, or any other suitable dimensions respectively.

A multileaf collimator of the type described with reference to Figure 12, 13 or 14 may be used as part of a multi-level MLC according to the disclosure. The multi-level MLC may include a first or bottom MLC and a second or top MLC comprising beam-blocking leaves. The bottom and top MLCs can be arranged such that the longitudinal moving direction of the beam-blocking leaves of the bottom MLC is generally parallel with the longitudinal moving direction of the beam-blocking leaves of the top MLC. The first and second MLCs can be arranged such that the beam-blocking leaves of the top MLC laterally offset the beam-blocking leaves of the bottom MLC e.g. by about half or a third of a leaf width.

Either of the top or bottom MLC may have the configuration as described with reference to Figure 12, 13 or 14. The other of the bottom or top MLC may, for example, have the configuration as shown in Figure 11, Figures 3A and 3B, or Figures 8A and 8B. Both of the top or bottom MLC may have the configuration as described with reference to Figure 12, 13 or 14.

The retraction or opening of one or more pairs of beam-blocking leaves in one layer of the MLC may create an opening that overlines or underlies the aperture 512, 514 and/or 524 in another layer of the MLC to control the size and shape of the radiation beam, enabling SRS delivery.

The leaves of the multi-layer MLC may be moved with respect to another to shape a radiation beam in a similar manner to that shown in Figure 1. By moving the leaves one layer of the MLC and relative to the leaves of another layer of the MLC such that any part of the aperture the aperture 512, 514 and/or 524 in one layer of the MLC is obscured by the leaves of another layer of the MLC, the apertures 512, 514 and/or 524 do not allow the passage of radiation through the multi-layer MLC at the apertures 512, 514 and/or 524, accomplishing a general functionality of the MLC - for example as shown in Figure 1. The MLC of the embodiment can therefore be used in a SRS delivery mode or a general MLC mode.

Various embodiments of MLCs enabling and enhancing radiosurgery or stereotactic radiosurgery are described. Advantageously, the "MLC cone" solution provided by this disclosure enables delivery of radiosurgery or stereotactic radiosurgery while preserving the broad scope or general functionality of the MLCs in shaping radiation beams for other applications by using measures such as additional blocking or treatment planning system. The "MLC cone" solution can deliver beam profiles generally comparable to those provided by conventional SRS cones. The efficiency of delivery is better than conventional "virtual cone" solution because double pass per couch angle would not be needed. The through-hole(s) in an MLC provided by this disclosure ensures consistent accuracy in delivery as compared to the conventional "virtual cone" approach which suffers from inaccurate delivery arising from the inconsistency in MLC positioning. The "MLC cone" solution provided by this disclosure can also appease the users who do not believe in the conventional "virtual cone" approach. The "MLC cone" solution is much more equivalent to SRS cone beam profiles and can produce more robust implementation requiring less quality assurance (QA).

Accordingly, a multileaf collimator is provided comprising a plurality of beam-blocking leaves arranged side by side in a first bank and a plurality of beam-blocking leaves arranged side by side in a second bank opposite to the first bank. At least one of the beam-blocking leaves in the first bank is provided with a first through-hole configured to allow a radiation beam to pass through for radiosurgery. The first through-hole may have a generally truncated cone shape or cylindrical shape. As used herein, a truncated cone refers to a result of cutting a cone by a plane parallel to the base and removing the part containing the apex.

In some embodiments, at least one of the beam-blocking leaves in the second bank is provided with a second through-hole configured to allow a radiation beam to pass through for radiosurgery. The second through-hole may have a size different from the size of the first through-hole. The second through-holes may have a generally truncated cone shape or cylindrical shape.

In some embodiments, the beam-blocking leaf provided with the first through-hole in the first bank may be further provided with a second through-hole configured to allow a radiation beam to pass through for radiosurgery. The second through-hole may have a size different from the size of the first through-hole.

An apparatus is provided comprising a first multileaf collimator including a plurality of pairs of beam-blocking leaves each comprising an end portion. The end portions of beam-blocking leaves of two adjacent pairs are configured to collectively form an aperture when the two adjacent pairs of beam-blocking leaves are closed. The aperture may have a generally circular shape in a beam's eye view.

In some embodiments, the end portions of the beam-blocking leaves of the two adjacent pairs may be configured to form the aperture having a generally truncated cone shape or cylindrical shape.

In some embodiments, the apparatus may further comprise a second multileaf collimator including a plurality of pairs of beam-blocking leaves. The first multileaf collimator may be arranged in a first level and the second multileaf collimator may be arranged in a second level. The beam-blocking leaves of the first multileaf collimator are longitudinal movable in a first direction and the beam-blocking leaves of the second multileaf collimator are longitudinal movable in a second direction generally parallel with the first direction. Each of the beam-blocking leaves of the second multileaf collimator laterally offsets a beam-blocking leaf of the first multileaf collimator in a beam's eye view.

In some embodiments, the beam-blocking leaves of the second multileaf collimator have a tip profile in the beam's eye view comprising a middle line section orthogonal to the second direction and a beveled line section at each side of the middle line section. The beam-blocking leaves may have a chamfer angle formed between the beveled line and the middle line ranging from 20-80 degrees.

In some embodiments, the first multileaf collimator includes, in addition to the beam-blocking leaves of the two adjacent pairs, beam-blocking leaves having a tip profile in the beam's eye view comprising a middle line section orthogonal to the first direction and a beveled line section at each side of the middle line section. The end portions of the beam-blocking leaves of the two adjacent pairs may have a tip profile in the beam's eye view comprising a middle line section orthogonal to the first direction, a beveled line section at a side of the middle line section, and a quarter-circular line section at another side of the middle line section.

A radiosurgery method using a multi-level multileaf collimator (MLC) is provided. The multi-level MLC comprises a first MLC having a plurality of pairs of beam-blocking leaves in a first level and a second MLC having a plurality of pairs of beam-blocking leaves in a second level. At least two adjacent pairs of beam-blocking leaves in the first MLC have end portions configured to collectively form a first aperture when the two adjacent pairs of beam-blocking leaves are closed. The method comprises opening a pair of beam-blocking leaves in the second MLC overlaying the at least two adjacent pairs of beam-blocking leaves of the first MLC to form a second aperture in the second MLC, where the second aperture in the second MLC has a size greater than the size of the first aperture in the first MLC; closing the plurality of pairs of beam-blocking leaves of the first MLC, whereby the beam-blocking leaves of the two adjacent pairs of the first MLC partially block the second aperture in the second MLC, allowing the first aperture in the first MLC to control the size and/or shape of a radiation beam passing through the multi-level MLC; and delivering the radiation beam to a target volume through the multi-level MLC, whereby the radiation beam delivered to the target volume is sized and shaped by the first aperture in the first MLC.

In some embodiments, the end portions of the beam-blocking leaves of the two adjacent pairs of the first MLC may be configured so that the first aperture formed has a generally rectangular shape in a beam's eye view. In some embodiments, the end portions of the beam-blocking leaves of the two adjacent pairs of the first MLC may be configured so that the first aperture formed may have a generally circular shape in a beam's eye view.

Various embodiments have been described with reference to the figures. It should be noted that some figures are not necessarily drawn to scale. The figures are only intended to facilitate the description of specific embodiments, and are not intended as an exhaustive description or as a limitation on the scope of the disclosure. Further, in the figures and description, specific details may be set forth in order to provide a thorough understanding of the disclosure. It will be apparent to one of ordinary skill in the art that some of these specific details may not be employed to practice embodiments of the disclosure. In other instances, well known components or process steps may not be shown or described in detail in order to avoid unnecessarily obscuring embodiments of the disclosure.

All technical and scientific terms used herein have the meaning as commonly understood by one of ordinary skill in the art unless specifically defined otherwise. As used in the description and appended claims, the singular forms of "a," "an," and "the" include plural references unless the context clearly dictates otherwise. The term "or" refers to a nonexclusive "or" unless the context clearly dictates otherwise. Further, the term "first" or "second" etc. may be used to distinguish one element from another in describing various similar elements. It should be noted the terms "first" and "second" as used herein include references to two or more than two. Further, the use of the term "first" or "second" should not be construed as in any particular order unless the context clearly dictates otherwise.

Various relative terms such as "upper," "above," "top," "over," "on," "below," "under," "bottom," "higher," "lower" or similar terms may be used herein for convenience in describing relative positions, directions, or spatial relationships in conjunction with the drawings. The use of the relative terms should not be construed as to imply a necessary positioning, orientation, or direction of the structures or portions thereof in manufacturing or use, and to limit the scope of the invention.

Various embodiments of radiosurgery methods, not forming part of the invention, are described in connection with the drawings. It will be appreciated that more or fewer steps, actions, or processes may be incorporated into the methods without departing from the scope of the disclosure. No particular order is implied by the steps described herein. It further will be appreciated that the methods described in conjunction with drawings may be embodied in machine-executable instructions (e.g. software). The instructions can be used to cause a general-purpose or special-purpose processor that is programmed with the instructions to perform the operations described. Alternatively, the operations might be performed by specific hardware components that contain hardwired logic for performing the operations, or by any combination of programmed computer components and custom hardware components. The methods may be provided as a computer program product that may include a machine-readable medium having stored thereon instructions that may be used to program a computer (or other electronic devices) to perform the methods. For the purposes of this specification, the term "machine-readable medium" shall be taken to include any medium that is capable of storing or encoding a sequence of instructions for execution by the machine and that causes the machine to perform any one of the methodologies of the present invention. The term "machine-readable medium" shall accordingly be taken to include, but not be limited to, solid-state memories, optical and magnetic disks. Furthermore, it is common in the art to speak of software, in one form or another (e.g., program, procedure, process, application, module, logic, etc.), as taking an action or causing a result. Such expressions are merely a shorthand way of saying that execution of the software by a computer causes the processor of the computer to perform an action or produce a result.

Various embodiments of radiosurgery methods and apparatuses have been described. Those skilled in the art will appreciate that various other modifications may be made. For example, in a multilevel MLC, an SRS aperture may be formed in the bottom MLC as described above in connection with FIGS. 4A-10C. Alternatively, or additionally, an SRS aperture may be formed in the top MLC. The invention is defined by the appended claims.

## Claims

1. An apparatus, comprising:
a first multileaf collimator (120, 320) having a plurality of pairs of longitudinally movable beam-blocking leaves (240a, 240b, 260a, 260b) arranged in opposing banks, wherein the beam-blocking leaves are each provided with an end portion;
wherein:
end portions of leaves of two adjacent pairs of leaves each have a non-planar tip profile from a beam's eye view comprising a recess that defines a space;
the spaces at the end portions (242, 244, 246) of leaves of the two adjacent pairs (240, 260) of the beam-blocking leaves (240a, 240b, 260a, 260b) are configured to collectively form an aperture (270) when the two adjacent pairs (240, 260) of beam-blocking leaves (240a, 240b, 260a, 260b) are closed; and
the aperture (270) has a generally circular shape in the beam's eye view.

2. The apparatus of claim 1 wherein at least one of the plurality of beam-blocking leaves (240a, 240b, 260a, 260b) is provided with a first through hole (512) configured to provide a radiation passage to allow a radiation beam (103) to pass through for radiosurgery.

3. The apparatus of claim 1 or 2, wherein the aperture has a generally truncated cone or cylindrical shape.

4. The apparatus of any one of claims 1 to 3, wherein at least one of the beam-blocking leaves (240a, 240b, 260a, 260b) has a tip profile in the beam's eye view comprising a middle line section (242) orthogonal to a direction in which the beam-blocking leaves (240a, 240b, 260a, 260b) are longitudinally moveable and a beveled line section (244) at at least one side of the middle line section (242);
and optionally wherein:
said at least one of the beam-blocking leaves (240a, 240b, 260a, 260b) has a chamfer angle formed between the beveled line (244) and the middle line (242) ranging from 20-80 degrees.

5. The apparatus of any one of claims 1 to 4, wherein at least one of the beam-blocking leaves (240a, 240b, 260a, 260b) of the two adjacent pairs (240, 260) of the first collimator (120, 320) have a tip profile in the beam's eye view comprising a middle line section (242) orthogonal to a direction in which the beam-blocking leaves (240a, 240b, 260a, 260b) are longitudinally moveable and a quarter-circular line section (246) at at least one side of the middle line section (242).

6. The apparatus of claim 2 wherein the first through-hole (512) is provided in a first bank of said beam-blocking leaves (240a, 240b, 260a, 260b) arranged side by side, the first bank being opposite a second bank of beam-blocking leaves arranged side by side;
and optionally wherein:
the first through-hole (512) has a generally truncated cone shape or cylindrical shape.

7. The apparatus of claim 6 wherein at least one of the plurality of beam-blocking leaves in the second bank is provided with a second through-hole (524) configured to allow a radiation beam to pass through for radiosurgery, wherein the second through-hole has a size different from a size of the first through-hole (512);
or:
wherein the at least one of the plurality of beam-blocking leaves in the first bank is further provided with a second through-hole (514) configured to allow a radiation beam to pass through for radiosurgery, wherein the second through-hole (514) has a size different from a size of the first through-hole (512).

8. The apparatus of claim 2, claim 6 or claim 7, wherein the through-hole (512, 514, 524) or the aperture (270) is disposed in or proximate to a middle of the plurality of beam blocking leaves (240a, 240b, 260a, 260b) the multileaf collimator to allow alignment of the through-hole (512, 514, 524) or the aperture (270) with a beam's (103) central axis (109) when in use;
and/or:
wherein the through-hole (512, 514, 524) or the aperture (270) has a projected diameter of a size at an isocenter plane (108) suitable for stereotactic radiosurgery, SRS.

9. The apparatus of any one of claims 1 to 8, further comprising a second multileaf collimator (130, 330) comprising a plurality of beam-blocking leaves (132, 332), wherein
the first multileaf collimator (120, 320) is arranged in a first level and the second multileaf collimator (130, 330) is arranged in a second level;
the beam-blocking leaves (122, 322) of the first multileaf collimator (120, 320) are longitudinally movable in a first direction, the beam-blocking leaves (132, 332) of the second multileaf collimator (130, 330) are longitudinally movable in a second direction generally parallel with the first direction; and
each of the beam-blocking leaves (132, 332) of the second multileaf collimator (130, 330) is laterally offset from a beam-blocking leaf (122, 322) of the first multileaf collimator (120, 320) in the beam's eye view.

10. The apparatus of claim 9, wherein at least one of the plurality of beam-blocking leaves (132, 332) of the second multileaf collimator (130, 330) is provided with at least one of (i) a through-hole (512, 514, 524) configured to provide a radiation passage to allow a radiation beam (103) to pass through for radiosurgery and (ii) an end portion that defines a space configured to provide an aperture (334) to allow a radiation beam (103) to pass through for radiosurgery.

11. The apparatus of claim 10, wherein the second multileaf collimator (130, 330) is configured like the first multileaf collimator (120, 320) as defined in any one of claims 1 to 8.

12. A non-medical method of controlling a multi-level multileaf collimator (110) including:
a first multileaf collimator (120, 320) having a plurality of pairs of longitudinally movable beam-blocking leaves (240a, 240b, 260a, 260b) arranged in opposing banks in a first level, wherein the beam-blocking leaves are each provided with an end portion; and
a second multileaf collimator (130, 330) having a plurality of beam-blocking leaves (132, 332) in a second level;
wherein in the first multileaf collimator (120, 320):
end portions of leaves of two adjacent pairs of leaves each have a non-planar tip profile from a beam's eye view comprising a recess that defines a space;
the spaces at the end portions (242, 244, 246) of leaves of the two adjacent pairs (240, 260) of the beam-blocking leaves (240a, 240b, 260a, 260b) are configured to collectively form an aperture (270) when the two adjacent pairs (240, 260) of beam-blocking leaves (240a, 240b, 260a, 260b) are closed,
wherein the aperture (270) has a generally circular shape in the beam's eye view;
the method comprising:
moving the beam-blocking leaves (240a, 240b, 260a, 260b) of the first (120, 320) and/or second multileaf collimator (130, 330) so that the beam-blocking leaves (132, 332) of the second multileaf collimator (130, 330) (i) selectively provide an opening between the leaves (132, 332) of the second multileaf collimator (130, 330) aligned with the space or (ii) selectively block the space.

13. The method of claim 12 wherein at least one of the plurality of beam-blocking leaves is provided with a first through hole (512, 514, 524).

14. The method of claim 12 or 13, further comprising:
opening a pair of beam-blocking leaves (132, 332) in the second multileaf collimator (130, 330) overlaying the at least two adjacent pairs (240, 260) of beam-blocking leaves (240a, 240b, 260a, 260b) of the first multileaf collimator (120, 320) to form a second aperture in the second multileaf collimator (130, 330), wherein the second aperture in the second multileaf collimator (130, 330) has a size greater than a size of the first aperture (270) in the first multileaf collimator (120, 320); and
closing the plurality of pairs of beam-blocking leaves (240a, 240b, 260a, 260b) of the first multileaf collimator (120, 320), wherein the beam-blocking leaves (240a, 240b, 260a, 260b) of the at least two adjacent pairs (240, 260) of the first multileaf collimator (120, 320) partially block the second aperture in the second multileaf collimator (130, 330, allowing the first aperture (270) in the first multileaf collimator (120, 320) to control a size of a radiation beam (103) passing through the multi-level multileaf collimator (110).

15. The method of claim 12, 13 or 14, further comprising aligning a center of the first aperture (270) in the first multileaf collimator (120, 320) with the radiation beam's central axis (109).

## Patentansprüche

1. Vorrichtung, umfassend:
einen ersten Mehrblattkollimator (120, 320), der eine Vielzahl von Paaren von längsverschiebbaren strahlblockierenden Blättern (240a, 240b, 260a, 260b) aufweist, die in gegenüberliegenden Reihen angeordnet sind, wobei jedes strahlblockierende Blatt mit einem Endabschnitt bereitgestellt ist;
wobei:
Endabschnitte von Blättern von zwei benachbarten Blattpaaren aus einer Strahlperspektive jeweils ein nichtplanares Spitzenprofil aufweisen, das eine Vertiefung umfasst, die einen Zwischenraum definiert;
die Zwischenräume an den Endabschnitten (242, 244, 246) von Blättern der zwei benachbarten Paare (240, 260) der strahlblockierenden Blätter (240a, 240b, 260a, 260b) so ausgelegt sind, dass sie gemeinsam eine Öffnung (270) ausbilden, wenn die zwei benachbarten Paare (240, 260) von strahlblockierenden Blättern (240a, 240b, 260a, 260b) geschlossen sind; und
die Öffnung (270) aus der Strahlperspektive eine im Allgemeinen kreisförmige Form aufweist.

2. Vorrichtung nach Anspruch 1, wobei mindestens eines von der Vielzahl von strahlblockierenden Blättern (240a, 240b, 260a, 260b) mit einer ersten Durchgangsbohrung (512) bereitgestellt ist, die so ausgelegt ist, dass sie einen Strahlungsdurchgang bereitstellt, um zu ermöglichen, dass ein Strahlenbündel (103) für die Radiochirurgie hindurchtritt.

3. Vorrichtung nach Anspruch 1 oder 2, wobei die erste Öffnung eine im Allgemeinen kegelstumpfartige oder zylindrische Form aufweist.

4. Vorrichtung nach einem der Ansprüche 1 bis 3, wobei mindestens eines von den strahlblockierenden Blättern (240a, 240b, 260a, 260b) aus der Strahlperspektive ein Spitzenprofil aufweist, das einen Mittellinienteilabschnitt (242) orthogonal zu einer Richtung, in der die strahlblockierenden Blätter (240a, 240b, 260a, 260b) längsverschiebbar sind, und einen Schräglinienteilabschnitt (244) an mindestens einer Seite des Mittellinienteilabschnitts (242) umfasst;
und wobei optional:
das mindestens eine von den strahlblockierenden Blättern (240a, 240b, 260a, 260b) einen Fasenwinkel, der zwischen der Schräglinie (244) und der Mittellinie (242) ausgebildet ist, im Bereich von 20-80 Grad aufweist.

5. Vorrichtung nach einem der Ansprüche 1 bis 4, wobei mindestens eines von den strahlblockierenden Blättern (240a, 240b, 260a, 260b) der zwei benachbarten Paare (240, 260) des ersten Kollimators (120, 320) aus der Strahlperspektive ein Spitzenprofil aufweist, das einen Mittellinienteilabschnitt (242) orthogonal zu einer Richtung, in der die strahlblockierenden Blätter (240a, 240b, 260a, 260b) längsverschiebbar sind, und einen Viertelkreislinienteilabschnitt (246) an mindestens einer Seite des Mittellinienteilabschnitts (242) umfasst.

6. Vorrichtung nach Anspruch 2, wobei die erste Durchgangsbohrung (512) in einer ersten Reihe von strahlblockierenden Blättern (240a, 240b, 260a, 260b) bereitgestellt ist, die nebeneinander angeordnet sind, wobei die erste Reihe einer zweiten Reihe von nebeneinander angeordneten strahlblockierenden Blättern gegenüberliegt;
und wobei optional:
die erste Durchgangsbohrung (512) eine im Allgemeinen kegelstumpfartige oder zylindrische Form aufweist.

7. Vorrichtung nach Anspruch 6, wobei mindestens eines von der Vielzahl von strahlblockierenden Blättern in der zweiten Reihe mit einer zweiten Durchgangsbohrung (524) bereitgestellt ist, die so ausgelegt ist, dass sie ermöglicht, dass ein Strahlenbündel für die Radiochirurgie hindurchtritt, wobei die zweite Durchgangsbohrung eine Größe aufweist, die sich von einer Größe der ersten Durchgangsbohrung (512) unterscheidet;
oder:
wobei das mindestens eine von der Vielzahl von strahlblockierenden Blättern in der ersten Reihe ferner mit einer zweiten Durchgangsbohrung (514) bereitgestellt ist, die so ausgelegt ist, dass sie ermöglicht, dass ein Strahlenbündel für die Radiochirurgie hindurchtritt, wobei die zweite Durchgangsbohrung (514) eine Größe aufweist, die sich von einer Größe der ersten Durchgangsbohrung (512) unterscheidet.

8. Vorrichtung nach Anspruch 2, 6 oder 7, wobei die Durchgangsbohrung (512, 514, 524) oder die Öffnung (270) in oder nahe einer Mitte der Vielzahl von strahlblockierenden Blättern (240a, 240b, 260a, 260b) des Mehrblattkollimators angeordnet ist, um bei Verwendung die Ausrichtung der Durchgangsbohrung (512, 514, 524) oder der Öffnung (270) mit der Mittelachse (109) eines Strahls (103) zu ermöglichen;
und/oder:
wobei die Durchgangsbohrung (512, 514, 524) oder die Öffnung (270) einen projizierten Durchmesser einer Größe in einer Isozentrumsebene (108) aufweist, der für die stereotaktische Radiochirurgie, SRS, geeignet ist.

9. Vorrichtung nach einem der Ansprüche 1 bis 8, ferner umfassend einen zweiten Mehrblattkollimator (130, 330), der eine Vielzahl von strahlblockierenden Blättern (132, 332) umfasst, wobei der erste Mehrblattkollimator (120, 320) auf einer ersten Stufe angeordnet ist und der zweite Mehrblattkollimator (130, 330) auf einer zweiten Stufe angeordnet ist;
die strahlblockierenden Blätter (122, 322) des ersten Mehrblattkollimators (120, 320) in einer ersten Richtung längsverschiebbar sind, die strahlblockierenden Blätter (132, 332) des zweiten Mehrblattkollimators (130, 330) in einer zweiten Richtung längsverschiebbar sind, die im Allgemeinen parallel zu der ersten Richtung verläuft; und
jedes der strahlblockierenden Blätter (132, 332) des zweiten Mehrblattkollimators (130, 330) aus der Strahlperspektive seitlich von einem strahlblockierenden Blatt (122, 322) des ersten Mehrblattkollimators (120, 320) versetzt ist.

10. Vorrichtung nach Anspruch 9, wobei mindestens eines von der Mehrzahl von strahlblockierenden Blättern (132, 332) des zweiten Mehrblattkollimators (130, 330) mit mindestens einem von (i) einer Durchgangsbohrung (512, 514, 524), die so ausgelegt ist, dass sie einen Strahlungsdurchgang ausbildet, um zu ermöglichen, dass ein Strahlenbündel (103) für die Radiochirurgie hindurchtritt, und (ii) einem Endabschnitt bereitgestellt ist, der einen Zwischenraum definiert, der so ausgelegt ist, dass er eine Öffnung (334) bereitstellt, um zu ermöglichen, dass ein Strahlenbündel (103) für die Radiochirurgie hindurchtritt.

11. Vorrichtung nach Anspruch 10, wobei der zweite Mehrblattkollimator (130, 330) wie der erste Mehrblattkollimator (120, 320) nach einem der Ansprüche 1 bis 8 ausgelegt ist.

12. Nichtmedizinisches Verfahren zur Steuerung eines mehrstufigen Mehrblattkollimators (110), einschließlich:
eines ersten Mehrblattkollimators (120, 320), der eine Vielzahl von Paaren von längsverschiebbaren strahlblockierenden Blättern (240a, 240b, 260a, 260b) aufweist, die in gegenüberliegenden Reihen auf einer ersten Stufe angeordnet sind, wobei jedes strahlblockierende Blatt mit einem Endabschnitt bereitgestellt ist; und
eines zweiten Mehrblattkollimators (130, 330), der eine Vielzahl von strahlblockierenden Blätter (132, 332) auf einer zweiten Stufe aufweist;
wobei im ersten Mehrblattkollimator (120, 320):
Endabschnitte von Blättern von zwei benachbarten Blattpaaren aus einer Strahlperspektive jeweils ein nichtplanares Spitzenprofil aufweisen, das eine Vertiefung umfasst, die einen Zwischenraum definiert;
die Zwischenräume an den Endabschnitten (242, 244, 246) der Blätter der zwei benachbarten Paare (240, 260) der strahlblockierenden Blätter (240a, 240b, 260a, 260b) so ausgelegt sind, dass sie gemeinsam eine Öffnung (270) ausbilden, wenn die zwei benachbarten Paare (240, 260) der strahlblockierenden Blätter (240a, 240b, 260a, 260b) geschlossen sind,
wobei die Öffnung (270) aus der Strahlperspektive eine im Allgemeinen kreisförmige Form aufweist;
das Verfahren Folgendes umfassend:
Verschieben der strahlblockierenden Blätter (240a, 240b, 260a, 260b) des ersten (120, 320) und/oder des zweiten Mehrblattkollimators (130, 330), so dass die strahlblockierenden Blätter (132, 332) des zweiten Mehrblattkollimators (130, 330) (i) selektiv eine Öffnung zwischen den Blättern (132, 332) des zweiten Mehrblattkollimators (130, 330) bereitstellen, die mit dem Zwischenraum ausgerichtet ist, oder (ii) selektiv den Zwischenraum blockieren.

13. Verfahren nach Anspruch 12, wobei mindestens eines von der Mehrzahl von strahlblockierenden Blättern mit einer ersten Durchgangsbohrung (512, 514, 524) bereitgestellt wird.

14. Verfahren nach Anspruch 12 oder 13, ferner umfassend:
Öffnen eines Paares von strahlblockierenden Blättern (132, 332) in dem zweiten Mehrblattkollimator (130, 330), die die mindestens zwei benachbarten Paare (240, 260) von strahlblockierenden Blättern (240a, 240b, 260a, 260b) des ersten Mehrblattkollimators (120, 320) überlagern, um eine zweite Öffnung in dem zweiten Mehrblattkollimator (130, 330) auszubilden, wobei die zweite Öffnung in dem zweiten Mehrblattkollimator (130, 330) eine Größe aufweist, die größer ist als eine Größe der ersten Öffnung (270) in dem ersten Mehrblattkollimator (120, 320); und
Schließen der Vielzahl von Paaren von strahlblockierenden Blättern (240a, 240b, 260a, 260b) des ersten Mehrblattkollimators (120, 320), wobei die strahlblockierenden Blätter (240a, 240b, 260a, 260b) der mindestens zwei benachbarten Paare (240, 260) des ersten Mehrblattkollimators (120, 320) die zweite Öffnung in dem zweiten Mehrblattkollimator (130, 330) teilweise blockieren, um zu ermöglichen, dass die erste Öffnung (270) in dem ersten Mehrblattkollimator (120, 320) eine Größe eines durch den mehrstufigen Mehrblattkollimator (110) hindurchtretenden Strahlenbündels (103) steuert.

15. Verfahren nach Anspruch 12, 13 oder 14, ferner umfassend Ausrichten eines Mittelpunkts der ersten Öffnung (270) in dem ersten Mehrblattkollimator (120, 320) mit der Mittelachse (109) des Strahlenbündels.

## Revendications

1. Appareil, comprenant :
un premier collimateur multilames (120, 320) ayant une pluralité de paires de lames bloquant le faisceau déplaçables longitudinalement (240a, 240b, 260a, 260b) disposées en rangées opposées, dans lequel les lames bloquant le faisceau sont chacune munies d'une partie d'extrémité ;
dans lequel :
les extrémités des lames de deux paires de lames adjacentes présentent chacune un profil de pointe non plan dans une vue du faisceau comprenant un renfoncement qui définit un espace ;
les espaces au niveau des parties d'extrémité (242, 244, 246) des lames des deux paires adjacentes (240, 260) des lames bloquant le faisceau (240a, 240b, 260a, 260b) sont configurés pour former collectivement une ouverture (270) lorsque les deux paires adjacentes (240, 260) de lames bloquant le faisceau (240a, 240b, 260a, 260b) sont fermées ; et
l'ouverture (270) a une forme généralement circulaire dans la vue du faisceau.

2. Appareil selon la revendication 1, dans lequel au moins l'une de la pluralité de lames bloquant le faisceau (240a, 240b, 260a, 260b) est munie d'un premier trou traversant (512) configuré pour fournir un passage de rayonnement permettant à un faisceau de rayonnement (103) de passer pour la radiochirurgie.

3. Appareil selon la revendication 1 ou 2, dans lequel l'ouverture a une forme généralement tronconique ou cylindrique.

4. Appareil selon l'une quelconque des revendications 1 à 3, dans lequel au moins l'une des lames bloquant le faisceau (240a, 240b, 260a, 260b) a un profil de pointe dans la vue du faisceau comprenant une section de ligne médiane (242) orthogonale à une direction dans laquelle les lames bloquant le faisceau (240a, 240b, 260a, 260b) sont déplaçables longitudinalement et une section de ligne biseautée (244) au niveau d'au moins un côté de la section de ligne médiane (242) ;
et éventuellement dans lequel :
ladite au moins une lame bloquant le faisceau (240a, 240b, 260a, 260b) a un angle de chanfrein formé entre la ligne biseautée (244) et la ligne médiane (242) allant de 20 à 80 degrés.

5. Appareil selon l'une quelconque des revendications 1 à 4, dans lequel au moins l'une des lames bloquant le faisceau (240a, 240b, 260a, 260b) des deux paires adjacentes (240, 260) du premier collimateur (120, 320) a un profil de pointe dans la vue du faisceau comprenant une section de ligne médiane (242) orthogonale à une direction dans laquelle les lames bloquant le faisceau (240a, 240b, 260a, 260b) sont déplaçables longitudinalement et une section de ligne en quart de cercle (246) au niveau d'au moins un côté de la section de ligne médiane (242).

6. Appareil selon la revendication 2, dans lequel le premier trou traversant (512) est prévu dans une première rangée desdites lames bloquant le faisceau (240a, 240b, 260a, 260b) disposées côte à côte, la première rangée étant opposée à une seconde rangée de lames bloquant le faisceau disposées côte à côte ;
et éventuellement dans lequel :
le premier trou traversant (512) a une forme généralement tronconique ou cylindrique.

7. Appareil selon la revendication 6, dans lequel au moins l'une de la pluralité de lames bloquant le faisceau de la seconde rangée est munie d'un second trou traversant (524) configuré pour permettre à un faisceau de rayonnement de passer pour la radiochirurgie, dans lequel le second trou traversant a une taille différente de la taille du premier trou traversant (512) ;
ou :
dans lequel l'au moins l'une de la pluralité de lames bloquant le faisceau de la première rangée est en outre munie d'un second trou traversant (514) configuré pour permettre à un faisceau de rayonnement de passer pour la radiochirurgie, dans lequel le second trou traversant (514) a une taille différente de la taille du premier trou traversant (512).

8. Appareil selon la revendication 2, la revendication 6 ou la revendication 7, dans lequel le trou traversant (512, 514, 524) ou l'ouverture (270) sont disposés dans ou à proximité d'un milieu de la pluralité de lames bloquant le faisceau (240a, 240b, 260a, 260b) du collimateur multilames pour permettre l'alignement du trou traversant (512, 514, 524) ou de l'ouverture (270) avec l'axe central (109) d'un faisceau (103) lors de l'utilisation ;
et/ou :
dans lequel le trou traversant (512, 514, 524) ou l'ouverture (270) a un diamètre projeté d'une taille au niveau d'un plan isocentrique (108) adaptée à la radiochirurgie stéréotaxique, SRS.

9. Appareil selon l'une quelconque des revendications 1 à 8, comprenant en outre un second collimateur multilames (130, 330) comprenant une pluralité de lames bloquant le faisceau (132, 332), dans lequel
le premier collimateur multilames (120, 320) est disposé dans un premier niveau et le second collimateur multilames (130, 330) est disposé dans un second niveau ;
les lames bloquant le faisceau (122, 322) du premier collimateur multilames (120, 320) sont déplaçables longitudinalement dans une première direction, les lames bloquant le faisceau (132, 332) du second collimateur multilames (130, 330) sont déplaçables longitudinalement dans une seconde direction généralement parallèle à la première direction ; et
chacune des lames bloquant le faisceau (132, 332) du second collimateur multilames (130, 330) est décalée latéralement par rapport à une feuille bloquant le faisceau (122, 322) du premier collimateur multilames (120, 320) dans la vue du faisceau.

10. Appareil selon la revendication 9, dans lequel au moins l'une de la pluralité de lames bloquant le faisceau (132, 332) du second collimateur multilames (130, 330) est munie d'au moins l'un des éléments suivants : (i) un trou traversant (512, 514, 524) configuré pour fournir un passage de rayonnement permettant à un faisceau de rayonnement (103) de passer pour la radiochirurgie et (ii) une partie d'extrémité qui définit un espace configuré pour fournir une ouverture (334) permettant à un faisceau de rayonnement (103) de passer pour la radiochirurgie.

11. Appareil selon la revendication 10, dans lequel le second collimateur multilames (130, 330) est configuré comme le premier collimateur multilames (120, 320) tel que défini dans l'une quelconque des revendications 1 à 8.

12. Procédé non médical de commande d'un collimateur multilames à plusieurs niveaux (110) comportant :
un premier collimateur multilames (120, 320) ayant une pluralité de paires de lames bloquant le faisceau déplaçables longitudinalement (240a, 240b, 260a, 260b) disposées en rangées opposées dans un premier niveau, dans lequel les lames bloquant le faisceau sont chacune munies d'une partie d'extrémité ; et
un second collimateur multilames (130, 330) ayant une pluralité de lames bloquant le faisceau (132, 332) dans un second niveau ;
dans lequel dans le premier collimateur multilames (120, 320) :
les extrémités des lames de deux paires de lames adjacentes présentent chacune un profil de pointe non plan dans une vue du faisceau comprenant un renfoncement qui définit un espace ;
les espaces au niveau des parties d'extrémité (242, 244, 246) des lames des deux paires adjacentes (240, 260) des lames bloquant le faisceau (240a, 240b, 260a, 260b) sont configurés pour former collectivement une ouverture (270) lorsque les deux paires adjacentes (240, 260) de lames bloquant le faisceau (240a, 240b, 260a, 260b) sont fermées,
dans lequel l'ouverture (270) a une forme généralement circulaire dans la vue du faisceau ;
le procédé comprenant :
le déplacement des lames bloquant le faisceau (240a, 240b, 260a, 260b) du premier (120, 320) et/ou du second collimateur multilames (130, 330) de sorte que les lames bloquant le faisceau (132, 332) du second collimateur multilames (130, 330) (i) fournissent sélectivement une ouverture entre les lames (132, 332) du second collimateur multilames (130, 330) alignées avec l'espace ou (ii) bloquent sélectivement l'espace.

13. Procédé selon la revendication 12, dans lequel au moins l'une de la pluralité de lames bloquant le faisceau est munie d'un premier trou traversant (512, 514, 524).

14. Procédé selon la revendication 12 ou 13, comprenant en outre :
l'ouverture d'une paire de lames bloquant le faisceau (132, 332) dans le second collimateur multilames (130, 330) chevauchant les au moins deux paires adjacentes (240, 260) de lames bloquant le faisceau (240a, 240b, 260a, 260b) du premier collimateur multilames (120, 320) pour former une seconde ouverture dans le second collimateur multilames (130, 330), dans lequel la seconde ouverture dans le second collimateur multilames (130, 330) a une taille supérieure à la taille de la première ouverture (270) dans le premier collimateur multilames (120, 320) ; et
la fermeture de la pluralité de paires de lames bloquant le faisceau (240a, 240b, 260a, 260b) du premier collimateur multilames (120, 320), dans lequel les lames bloquant le faisceau (240a, 240b, 260a, 260b) des au moins deux paires adjacentes (240, 260) du premier collimateur multilames (120, 320) bloquent partiellement la seconde ouverture dans le second collimateur multilames (130, 330), permettant à la première ouverture (270) dans le premier collimateur multilames (120, 320) de contrôler la taille d'un faisceau de rayonnement (103) passant à travers le collimateur multilames à plusieurs niveaux (110).

15. Procédé selon la revendication 12, 13 ou 14, comprenant en outre l'alignement du centre de la première ouverture (270) dans le premier collimateur multilames (120, 320) avec l'axe central du faisceau de rayonnement (109).
